# EUROPEAN PATENT APPLICATION

(11) **EP 4 821 457 A1**
(43) Date of publication of application: **14.10.2026**
(21) Application number: 26167449.3
(22) Date of filing: 25.03.2026
(51) Int. Cl.: A61B 34/30, A61B 17/16, A61B 34/00, A61B 90/00

(54) **ROBOTIC SURGICAL SYSTEM, METHOD FOR CONTROLLING ROBOTIC SURGICAL SYSTEM, PROGRAM, AND STORAGE MEDIUM**

(30) Priority: 07.04.2025 JP 2025063190
(71) Applicant: KAWASAKI JUKOGYO KABUSHIKI KAISHA, Kobe-shi, Hyogo 650-8670 (JP)
(72) Inventor: MIZOHATA, Yuichi, Kobe-shi, Hyogo, 650-8670 (JP)
(74) Representative: Hoffmann Eitle

(57) **Abstract**

A robotic surgical system according to this disclosure includes a controller configured to execute processing of displaying, on a display, a graphical user interface including a distance indication representing a distance from a preset reference point to a predetermined portion of a surgical instrument while the surgical instrument is being moved to a desired position by an operation input.

## Description

### BACKGROUND OF THE INVENTION

### Field of the Invention

The present disclosure relates to a robotic surgical system, a method for controlling a robotic surgical system, a program, and a storage medium.

### Description of the Background Art

A robotic surgical system including a robot arm to which a surgical instrument is attached is known in the art. Japanese Patent Publication No. JP 7171647 discloses a surgical robot including a plurality of robot arms to each of which a surgical instrument is attached at a distal end, and an operation input that is attached to the robot arms and operates the robot arms. In this surgical robot, the operation input includes a pivot position teaching button for causing a storage to store a pivot position that is a pivot point of movement of the surgical instrument, which is attached to the robot arm. When the robot arms are operated by the operation input so that a distal end of the surgical instrument attached to a distal end side of each robot arm is moved to an insertion position of a trocar inserted through a body surface into a body of a patient, and the pivot position teaching button is pressed, a distal end position of the surgical instrument is stored in the storage as the pivot position. Further, the pivot position is set for each of the plurality of robot arms.

In the robotic surgical system disclosed in Japanese Patent No. 7171647, when a distal end of a surgical instrument attached to a distal end side of a robot arm is moved to an insertion position of a trocar inserted through a body surface into a body of a patient, and the pivot position teaching button is pressed, a distal end position of the surgical instrument is stored in the storage as the pivot position. Here, the trocar has a cylindrical shape, and the pivot position to be set differs depending on whether the distal end of the surgical instrument is moved to one side or to the other side of an outer surface of the cylindrical trocar. In this case, the distance between pivot positions set for the plurality of robot arms may become larger or smaller than a desired distance. Accordingly, it is desirable to appropriately specify the pivot positions of the surgical instruments attached to the plurality of robot arms.

### SUMMARY OF THE INVENTION

The present disclosure provides a robotic surgical system, a method for controlling a robotic surgical system, and a program that can appropriately specify pivot positions of surgical instruments attached to a plurality of robot arms.

A robotic surgical system according to a first aspect of the present disclosure includes a plurality of robot arms each having a distal end to which a surgical instrument is attached; an operation input configured to operate movement of the surgical instrument by the robot arms; a pivot-position specifier configured to store, in a storage, a pivot position serving as a pivot point for movement of the surgical instrument when operated after a distal end of the surgical instrument has been moved to a desired position by the operation input; and a controller configured to execute processing of displaying, on a display, a graphical user interface including a distance indication representing a distance from a preset reference point to a predetermined portion of the surgical instrument while the surgical instrument is being moved to the desired position by the operation input.

The robotic surgical system according to the first aspect of the present disclosure includes a controller configured to execute processing of displaying, on a display, a graphical user interface including a distance indication representing a distance from a preset reference point to a predetermined portion of the surgical instrument while the surgical instrument is being moved to the desired position by the operation input. Accordingly, while the surgical instrument is being moved to the desired position by the operation input, a distance indication representing the distance from the preset reference point to the predetermined portion of each surgical instrument is displayed, so that an operator can recognize the distance from the reference point to the predetermined portion of each surgical instrument. That is, the operator can specify the pivot position based on the distance from the reference point. As a result, pivot positions of the surgical instruments respectively attached to the plurality of robot arms can be appropriately specified.

A control method according to a second aspect of the present disclosure is a method for controlling a robotic surgical system including a plurality of robot arms each having a distal end to which a surgical instrument is attached, an operation input configured to operate movement of the surgical instrument by the robot arms, and a pivot-position specifier configured to store, in a storage, a pivot position serving as a pivot point for movement of the surgical instrument when operated after a distal end of the surgical instrument has been moved to a desired position by the operation input, the method including receiving an operation for moving the surgical instrument by the operation input; and displaying, on a display, a graphical user interface including a distance indication representing a distance from a preset reference point to a predetermined portion of the surgical instrument while the surgical instrument is being moved to the desired position by the operation input.

The method for controlling a robotic surgical system according to the second aspect of the present disclosure includes displaying, on a display, a graphical user interface including a distance indication representing a distance from a preset reference point to a predetermined portion of the surgical instrument while the surgical instrument is being moved to the desired position by the operation input. Accordingly, while the surgical instrument is being moved to the desired position by the operation input, a distance indication representing the distance from the preset reference point to the predetermined portion of each surgical instrument is displayed, so that an operator can recognize the distance from the reference point to the predetermined portion of each surgical instrument. That is, the operator can specify the pivot position based on the distance from the reference point. As a result, it is possible to provide a method for controlling a robotic surgical system that can appropriately specify pivot positions of surgical instruments respectively attached to a plurality of robot arms.

A program according to a third aspect of the present disclosure is a program for the method for controlling a robotic surgical system according to the second aspect. Accordingly, similar to the method for controlling a robotic surgical system according to the second aspect, it is possible to provide a program that can appropriately specify pivot positions of surgical instruments respectively attached to a plurality of robot arms.

A storage medium according to a fourth aspect of the present disclosure is a storage medium storing the program according to the third aspect. Accordingly, similar to the program according to the third aspect, it is possible to provide a storage medium storing the program that can appropriately specify pivot positions of surgical instruments respectively attached to a plurality of robot arms.

According to the present disclosure, pivot positions of surgical instruments respectively attached to a plurality of robot arms can be appropriately specified.

### BRIEF DESCRIPTION OF THE DRAWINGS

FIG. 1 is a view showing a configuration of a robotic surgical system according to one embodiment.
FIG. 2 is a view showing a display of a medical cart according to one embodiment.
FIG. 3 is a view showing a configuration of the medical cart according to one embodiment.
FIG. 4 is a view showing a configuration of a robot arm according to one embodiment.
FIG. 5 is a view showing an instrument.
FIG. 6 is a perspective view showing a configuration of an arm operation input according to one embodiment.
FIG. 7 is a view illustrating translational movement of the robot arm.
FIG. 8 is a view illustrating rotational movement of the robot arm.
FIG. 9 is a view showing an endoscope.
FIG. 10 is a view showing a pivot-position specifying tool.
FIG. 11 is a view showing an operation input according to one embodiment.
FIG. 12 is a view showing a right-hand side wrist portion according to one embodiment.
FIG. 13 is a view showing a left-hand side wrist portion according to one embodiment.
FIG. 14 is a perspective view showing foot pedals according to one embodiment.
FIG. 15 is a control block diagram of the robotic surgical system according to one embodiment.
FIG. 16 is a control block diagram of the robot arm according to one embodiment.
FIG. 17 is a control block diagram of a positioner and the medical cart according to one embodiment.
FIG. 18 is a control block diagram of the operation input according to one embodiment.
FIG. 19 is a flowchart illustrating a method for controlling the robotic surgical system according to one embodiment.
FIG. 20 is a view illustrating how to specify a pivot position.
FIG. 21 is a view showing a pivot distance indication superimposed on an image captured by the endoscope.
FIG. 22 is a view showing a pivot distance indication in a state in which a pivot position of the endoscope is specified.
FIG. 23 is a view showing a pivot distance indication in a state in which a distance between the pivot position of the endoscope and a distal end of one pivot-position specifying tool is indicated.
FIG. 24 is a view showing a pivot distance indication in a state in which the pivot position of the endoscope and pivot positions of all instruments are specified.

### DESCRIPTION OF THE PREFERRED EMBODIMENT

### (Configuration of Robotic Surgical System)

The following description describes a configuration of a robotic surgical system 500 according to this embodiment. The robotic surgical system 500 includes a surgical robot 100, a remote operation apparatus 200, a vision unit 300 and an image processing unit 400. Here, the remote operation apparatus 200 is an example of an operation apparatus.

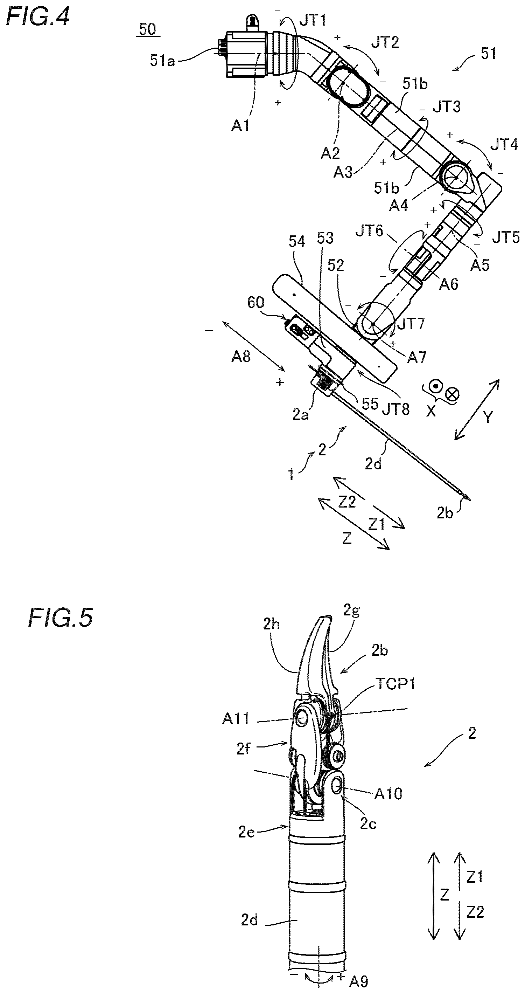

In this specification, a longitudinal direction of a surgical instrument 1 is defined as a Z direction as shown in FIG. 4. A distal end of the surgical instrument 1 is defined as a Z1 side, and a proximal end of the surgical instrument 1 is defined as a Z2 side. A direction perpendicular to the Z direction is defined as an X direction. A direction perpendicular to the Z direction and the X direction is defined as a Y direction.

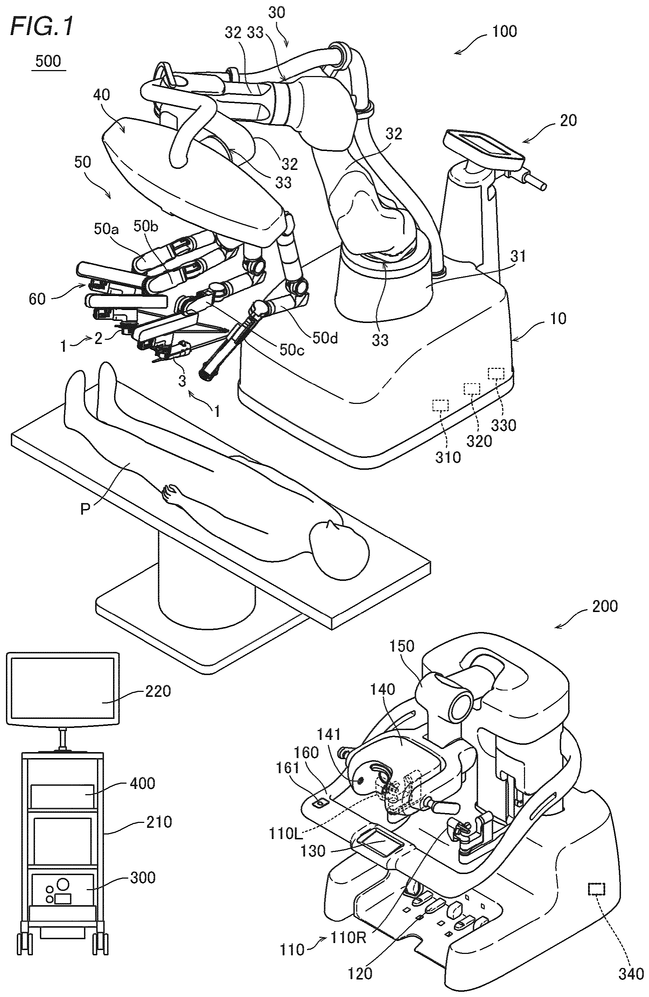

As shown in FIG. 1, the surgical robot 100 is arranged in an operating room. The remote operation apparatus 200 is located remote from the surgical robot 100. Also, the remote operation apparatus 200 is configured to receive instructions as to the surgical instruments 1. Specifically, an operator, such as a doctor, can provide the remote operation apparatus 200 with an instruction indicating a desired motion of the surgical robot 100. The remote operation apparatus 200 transmits the provided command to the surgical robot 100. The surgical robot 100 is configured to perform the motion in accordance with the command received. The surgical robot 100 is arranged in the operating room, which is a sterile field.

### (Configuration of Surgical Robot)

As shown in FIG. 1, the surgical robot 100 includes a medical cart 10, a cart positioner operation input 20, a positioner 30, an arm base 40, a plurality of robot arms 50 and arm operation inputs 60 provided in the robot arms 50. Here, the arm operation input 60 is an example of an operation input.

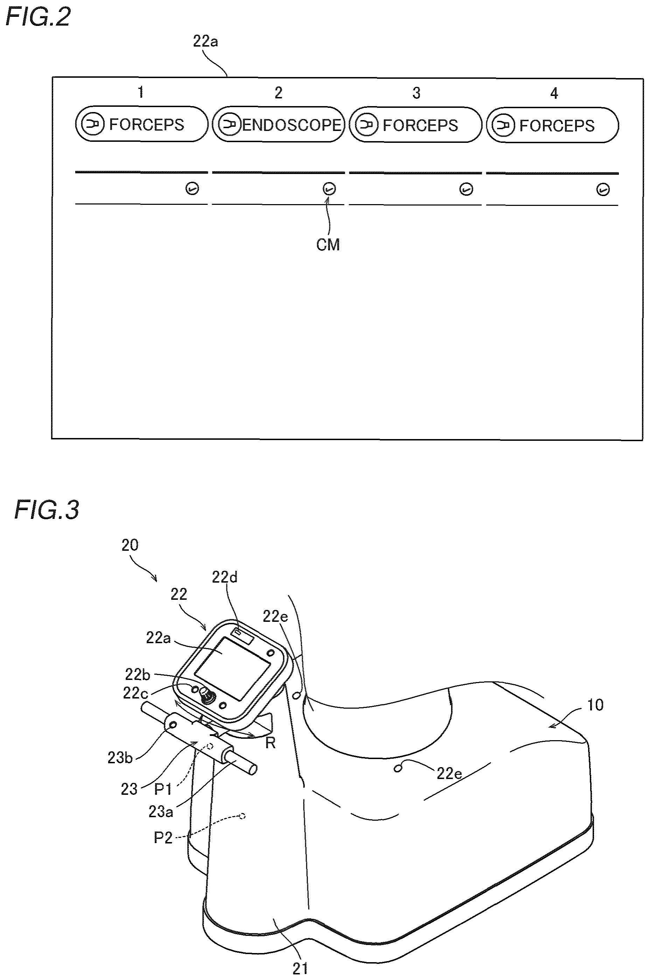

As shown in FIG. 3, the cart positioner operation input 20 is arranged in a rear part of the medical cart 10 and supported by a cart positioner operation support 21, and the medical cart 10 or the positioner 30 can be moved in accordance with a manual operation of the cart positioner operation input 20. The cart positioner operation input 20 includes the input device 22 and an operating handle 23. The input device 22 is configured to receive instructions to move or change orientations of the positioner 30, the arm base 40 and the plurality of robot arms 50 to prepare a surgical operation mainly before the operation is carried out. The medical cart 10 includes the operation handle 23.

As shown in FIG. 3, the input device 22 includes the display 22a, a joystick 22b, an enable switch 22c, an error reset button 22d and speakers 22e. For example, the display 22a is a liquid crystal panel. As shown in FIG. 2, the display 22a indicates numbers corresponding to the plurality of robot arms 50. Also, the display 22a indicates types of surgical instruments 1 attached to the plurality of robot arms 50. The display 22a indicates checkmarks CM representing that their pivot positions PP (discussed later) have been set.

As shown in FIG. 3, the joystick 22b is arranged near the display 22a of the input device 22. When an operation mode displayed on the display 22a is selected, the positioner 30 can be three-dimensionally moved by operating the joystick 22b.

The enable switch 22c is arranged near the joystick 22b. The enable switch 22c is configured to enable or disable movement of the positioner 30. When the enable switch 22c is pressed so that movement of the positioner 30 is enabled, the positioner 30 can be moved in accordance with a manual operation of the joystick 22b.

The error reset button 22d is configured to reset an error of the robotic surgical system 500. An exemplary error is an error of abnormal deviation. The speakers 22e are a pair of speakers. The pair of speakers 22e are arranged at a position in the medical cart 10 near the positioner 30.

Also, the operation handle 23 is arranged near the display 22a. The operating handle 23 includes a throttle grip 23a that is configured to be gripped and twisted by an operator such as a nurse and an engineer to control movement of the medical cart 10. Specifically, the operating handle 23 is arranged under the input device 22. The medical cart 10 can move forward when the throttle grip 23a is twisted from a near side toward a far side. The medical cart 10 can move backward when the throttle grip 23a is twisted from the far side toward the near side. A speed of the medical cart 10 can be changed in accordance with a twisting amount of the throttle grip 23a. In addition, the operating handle 23 is configured to swing leftward and rightward as shown by an R direction, and to rotate the medical cart 10 depending on the swinging operation of the operating handle 23.

Also, the operation handle 23 includes an enable switch 23b configured to enable or disable movement of the medical cart 10. When the enable switch 23b is pressed so that movement of the medical cart 10 is enabled, the medical cart 10 can be moved in accordance with a manual operation of the throttle grip 23a of the operating handle 23.

For example, as shown in FIG. 1, the positioner 30 is constructed of a 7-axis multi-joint robot. The positioner 30 is arranged on the medical cart 10. The positioner 30 is configured to adjust a position of the arm base 40. The positioner 30 can three-dimensionally move the position of the arm base 40.

The positioner 30 includes a base 31, and a plurality of links 32 coupled to the base 31. The links 32 are coupled to each other by joints 33.

The arm base 40 is attached to a distal end of the positioner 30. In the plurality of robot arms 50, the proximal end of each robot arm 50 is attached to the arm base 40. The plurality of robot arms 50 are foldable into a storage posture. The arm base 40 and the plurality of robot arms 50 are covered by sterile drapes when used. The robot arms 50 are configured to support surgical instruments 1.

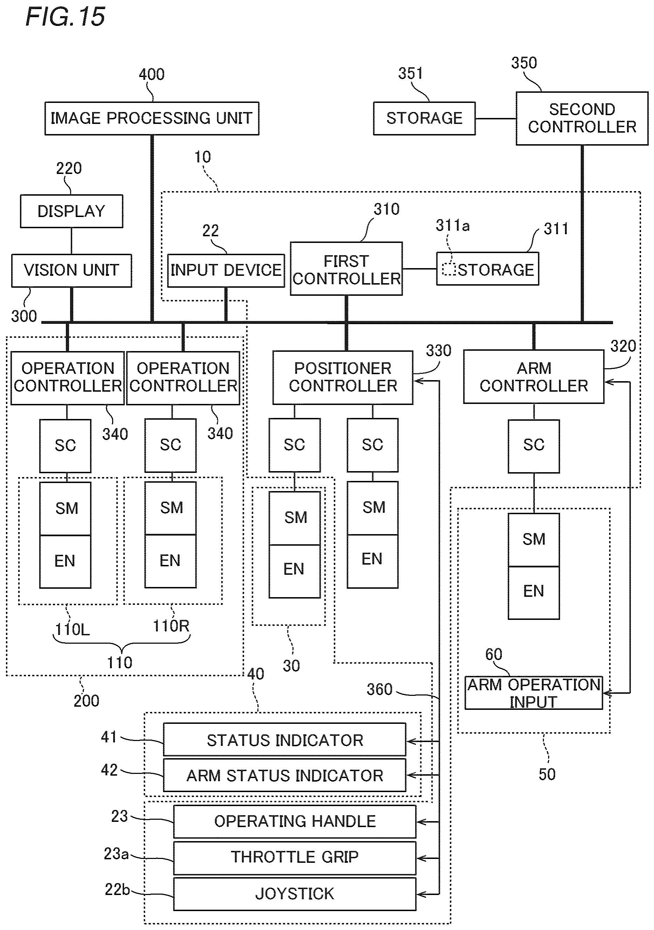

A status indicator 41 and an arm status indicator 42 shown in FIG. 15 are provided in the arm base 40. The status indicator 41 is configured to indicate a status of robotic surgical system 500. The arm status indicator 42 is configured to indicate states of robot arms 50.

Two or more robot arms 50 are provided as a plurality of robot arms. Specifically, four robot arms 50a, 50b, 50c and 50d are provided. The robot arms 50a, 50b, 50c and 50d have a similar configuration to each other.

As shown in FIG. 4, each robot arm 50 includes an arm 51, a first link 52, a second link 53, and a translation mechanism 54. The robot arm 50 includes joints JT1, JT2, JT3, JT4, JT5, JT6, JT7 and JT8. The joints JT1, JT2, JT3, JT4, JT5, JT6 and JT7 have A1, A2, A3, A4, A5, A6 and A7 axes as their rotation axes. JT8 has an A8 axis as its linear-motion axis. The arm 51 includes a base 51a and a link 51b.

The arm 51 is constructed of a 7-axis multi-joint robot arm. The first link 52 is arranged in a distal end of arm 51. The arm operation input 60 discussed later is attached to the second link 53. The translation mechanism 54 is arranged between the first link 52 and the second link 53. The second link 53 includes a holder 55 configured to hold the surgical instrument 1. The translation mechanism 54 is configured to translationally move the holder 55 to which the surgical instrument 1 is attached between a first position and a second position. The first position is a position of a Z2-direction side end of a moving range of the holder 55 moved by the translation mechanism 54 along the A8 axis. The second position is a position of a Z1-direction side end of the moving range of the holder 55 moved by the translation mechanism 54 along the A8 axis.

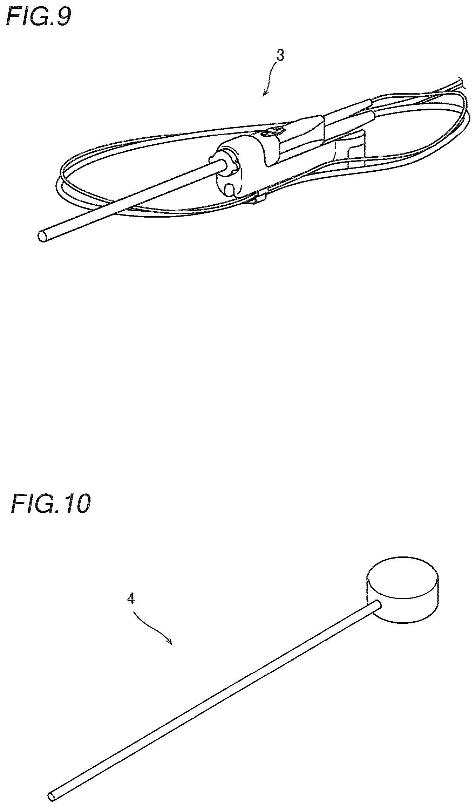

Surgical instruments 1 can be attached to the distal ends of the plurality of robot arms 50. The surgical instruments 1 include, for example, replaceable instruments 2, an endoscope 3 (see FIG. 9) configured to capture images of a part to be operated, a pivot-position specifying tool 4 (see FIG. 10) to set a pivot position PP described below, and the like. Each of the instruments 2 includes a driven unit 2a, an end effector 2b, a wrist joint 2c shown in FIG. 5, and a shaft 2d. The end effector 2b is connected to a distal end of the shaft 2d via the wrist joint 2c.

As shown in FIG. 1, an endoscope 3 is attached to the distal end of one, e.g., the robot arm 50c of the robot arms 50, and the instruments 2 are attached to the distal ends of the others, e.g., the robot arms 50a, 50b and 50d. The robot arms 50b and 50d are arranged adjacent to the robot arm 50c. The robot arm 50a is arranged adjacent to the robot arm 50b. The endoscope 3 is preferably attached to one of two robot arms 50b and 50c, which are located in a central part, of the four robot arms 50 arranged adjacent to each other. Here, the robot arm 50a is an example of a second robot arm and an end robot arm. In addition, the robot arms 50b and 50d are examples of the second robot arm and an adjacent robot arm. The robot arm 50c is an example of a first robot arm.

### (Configuration of Instrument)

As shown in FIG. 5, the end effector 2b, which includes jaw members 2g and 2h for example, is attached to the distal end of the instrument 2. Scissors, a grasper, a needle holder, a microdissector, a staple applier, a tucker, a vacuum cleaning tool, a snare wire, a clip applier, or the like can be used as the end effector 2b.

The instrument 2 includes a first support member 2e and a second support member 2f. The first support member 2e is attached to the shaft 2d. The second support member 2f is rotatably supported by the first support member 2e about an A10 axis, and rotatably supports the end effector 2b about an A11 axis, which intersects the A10 axis. The shaft 2d rotates about an A9 axis. The wrist joint 2c is arranged between the second support member 2f and the first support member 2e and rotatable about the A10 axis as an axis of rotation.

### (Configuration of Arm Operation Input)

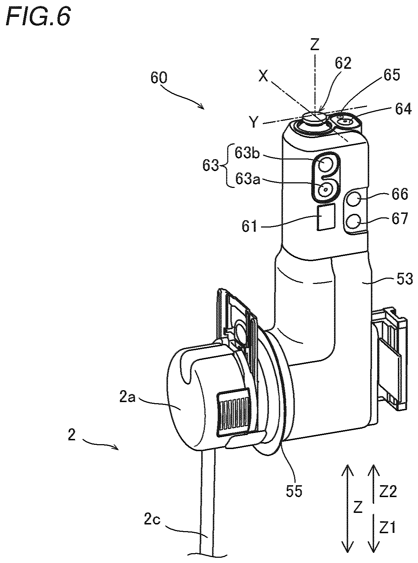

As shown in FIG. 6, the arm operation input 60 is mounted to the robot arm 50, and is configured to operate the robot arm 50. Specifically, the arm operation input 60 is mounted to the second link 53.

The arm operation input 60 includes an enable switch 61, a joystick 62, linear switches 63, a mode switching button 64, a mode indicator 65, a pivot button 66, and an adjustment button 67. The pivot button 66 is an example of a pivot-position specifier.

The enable switch 61 is configured to enable or disable movement of the robot arm 50 by means of the joystick 62 and the linear switches 63 when pressed. Movement of the surgical instrument 1 by the robot arm 50 is enabled when the enable switch 61 is pressed while the arm operation input 60 is grasped by an operator such as nurse, or assistant.

The joystick 62 is an operation tool configured to control movement of the surgical instrument 1 by the robot arm 50. The joystick 62 is an operation tool configured to control a moving direction and a moving speed of the robot arm 50. The robot arm 50 can be moved in accordance with a tilting direction and a tilting angle of the joystick 62.

The linear switches 63 are switches for moving the surgical instrument 1 in the Z direction, which is a longitudinal direction of the surgical instrument 1. The linear switches 63 include a linear switch 63a for moving the surgical instrument 1 in a direction in which the surgical instrument 1 is inserted into a patient P, and a linear switch 63b for moving the surgical instrument 1 in a direction in which the surgical instrument 1 is moved away from the patient P. The linear switch 63a and the linear switch 63b are constructed of a press-button switch.

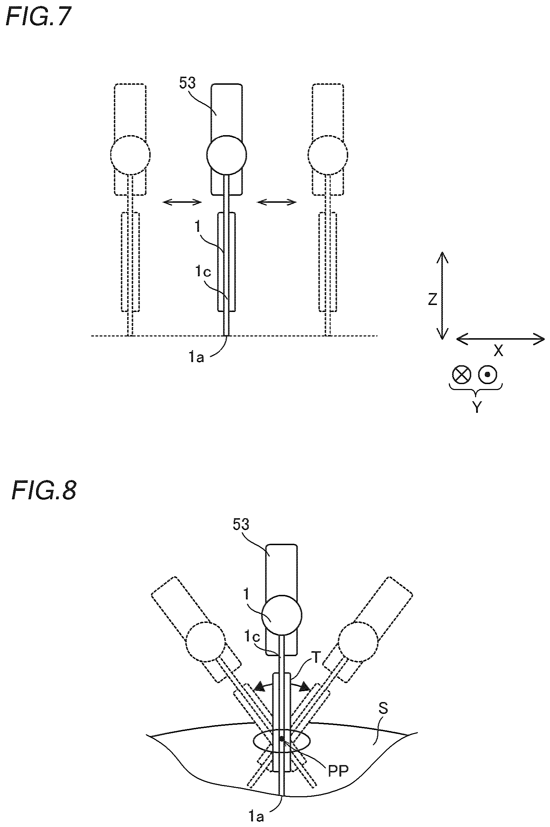

The mode switching button 64 is a press-button switch for switching between a translation mode in which the surgical instrument 1 is translationally moved, and a rotation mode in which the surgical instrument 1 is rotated. As shown in FIG. 7, in the translation mode in which the robot arm 50 is translationally moved, the robot arm 50 can be moved so that the distal end 1a of the surgical instrument 1 can be moved in an X-Y plane. As shown in FIG. 8, in the rotation mode in which the robot arm 50 is rotated, in a case in which any pivot position PP is not stored in the storage 351 shown in Fig. 15, the robot arm 50 can be moved so that the end effector 2b can be rotated about a center of the end effector 2b of the instrument 2 as the surgical instrument 1 on the A11 axis or the distal end of the end effector 2b as a pivot point, and in a case in which a pivot position PP is stored in the storage 351, the robot arm 50 can be moved so that the surgical instrument 1 can be rotated about the pivot position PP as a pivot point. In this case, the surgical instrument 1 is rotated with the shaft 1c of the surgical instrument 1 being inserted into a trocar T. The mode switching button 64 is arranged on a surface on a Z-direction side of the arm operation input 60.

The mode indicator 65 is configured to indicate which mode is selected. The mode indicator 65 is configured to light on to indicate the rotation mode, and to turn off indicate the translation mode. The mode indicator 65 also serves as a pivot position indicator to indicate that the pivot position PP is set. The mode indicator 65 is arranged on the surface on the Z-direction side of the arm operation input 60.

The pivot button 66 is a press-button switch configured to set the pivot position PP, which corresponds to the pivot point of the surgical instrument 1 attached to the robot arm 50. When the pivot button 66 is pressed, the pivot position PP is stored in the storage 351.

The adjustment button 67 is a button configured to optimize a position of the robot arm 50. After the pivot position PP is set with respect to the robot arm 50 to which the endoscope 3 is attached, when the adjustment button 67 is pressed, positions of the other robot arms 50 and the arm base 40 are optimized. The adjustment button 67 is a button different from the enable switch 61.

### (Remote Operation Apparatus)

For example, as shown in FIG. 1, the remote operation apparatus 200 is arranged in an operating room or outside the operating room. The remote operation apparatus 200 includes an operation input 110, foot pedals 120, a touch panel 130, a monitor 140, a support arm 150, a support bar 160, and an error reset button 161. The operation input 110 serves as a handle for operation that is configured to receive commands from an operator such as a doctor. Here, the monitor 140 is an example of a first display.

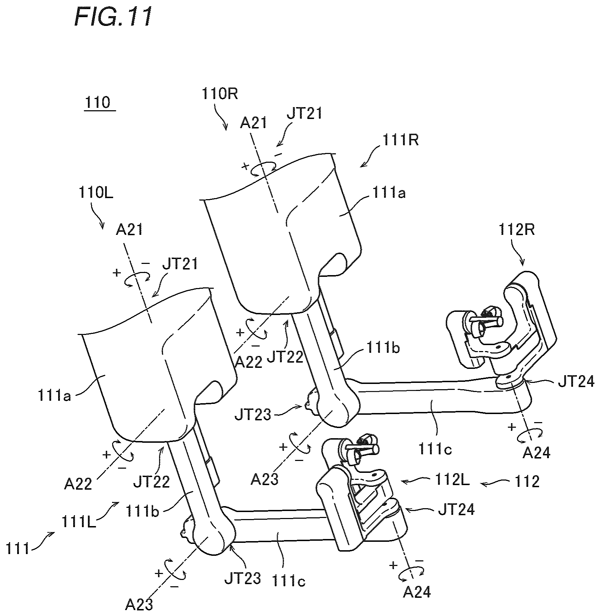

As shown in FIG. 11, the operation inputs 110 are handles configured to manipulate the surgical instrument 1. Also, the operation input 110 is configured to receive instructions for the surgical instruments 1. The operation input 110 includes an operation input 110L that is arranged on a left side from viewpoint of the operator such as a doctor and is configured to be manually operated by the operator's left hand, and an operation input 110R that is arranged on a right side from viewpoint of the operator and is configured to be manually operated by the operator's right hand. The operation inputs 110 include arm portions 111 and wrist portions 112. The operation input 110R includes an arm portion 111R and a wrist portion 112R. The operation input 110L includes an arm portion 111L and a wrist portion 112L.

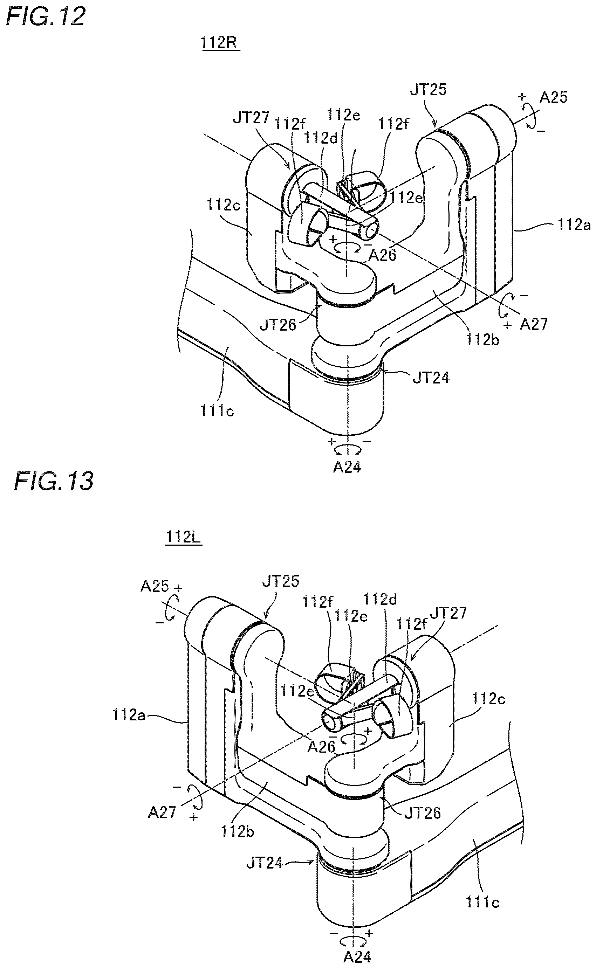

The arm portions 111 include joints JT21, JT22 and JT23 shown in FIG. 11, and joints JT24, JT25, JT26 and JT27 shown in FIGS. 12 and 13. The joints JT21, JT22, JT23, JT24, JT25, JT26 and JT27 have axes A21, A22, A23, A24, A25, A26 and A27.

As shown in FIG. 11, the arm portion 111R includes a link 111a, a link 111b and a link 111c. An upper end side of the link 111a is attached to the remote operation apparatus 200 rotatably about the A21 axis extending in a vertical direction. An upper end side of the link 111b is attached to a lower part of the link 111a rotatably about the A22 rotation axis extending in a horizontal direction. One end side of the link 111c is attached to a lower part of the link 111b rotatably about the A23 rotation axis extending in a horizontal direction. The wrist portion 112 is attached to another end side of the link 111c rotatably about the A24 rotation axis. The link 111a is connected to the remote operation apparatus 200 by the joint JT21. The link 111a is connected to the link 111b by the joint JT22. The link 111b is connected to the link 111c by the joint JT23. The arm portion 111 supports the wrist portion 112. Here, the arm portion 111L has a configuration similar to the arm portion 111R.

The wrist portions 112 include a wrist portion 112R shown in FIG. 12 operated by the operator's right hand, and a wrist portion 112L shown in FIG. 13 operated by the operator's left hand. A reference posture of the operation input 110R is shown in FIG. 12, and a reference posture of the operation input 110L is shown in FIG. 13. A configuration of the wrist portion 112R is similar to the wrist portion 112L.

The wrist portion 112 includes a link 112a, a link 112b, a link 112c, and a grip support 112d configured to be operated by an operator such as a doctor. The link 112a includes a proximal end connected to a distal end of the arm portion 111, and is configured to rotate about the A24 axis. The link 112b includes a proximal end connected to a distal end of the link 112a with respect to link 112a, and is configured to rotate about the A25 axis. The link 112c includes a proximal end connected to a distal end of the link 112b and a distal end to which the grip support 112d is connected, and is configured to rotate relative to the link 112b about the A26 axis. The grip support 112d rotates about the A27 axis relative to the link 112c. The link 112a, the link 112b and the link 112c have L shapes.

Each wrist portion 112 includes a pair of grips 112e operated by the operator to be opened and closed. The grips 112e are formed of thin plate-shaped levers, and near-side ends of the pair of grips 112e are rotatably coupled to a near-side end of the grip support 112d. The grips 112e include cylindrical finger insertion sections 112f. The operator can insert his or her fingers into the finger insertion sections 112f, and operate the wrist portion 112. Proximal ends of the pair of grips 112e are coupled to the grip support 112d so that an opening angle between the jaw 2g and the jaw 2h can be changed by increasing/decreasing an angle between the pair of grips 112e. One of the grips 112e includes a magnet, while the grip support 112d includes a Hall sensor. The magnet and the Hall sensor function as an angle detection sensor, and can provide an opening angle when the operator opens/closes the grips 112e. Here, the grip 112e may include a Hall sensor, while the grip support 112d may include a magnet.

As shown in FIG. 1, the monitor 140 is a scope-type display device configured to display images captured by the endoscope 3. The monitor 140 includes an information producer 141. The information producer 141 is configured to produce an error sound. The support arm 150 supports the monitor 140, and can adjust a height of the monitor 140 to a height of eyes of the operator such as doctor. The touch panel 130 is arranged on the support bar 160. When a head of the operator is detected by a sensor arranged near the monitor 140, the surgical robot 100 can receive manual operations from the remote operation apparatus 200. The operator will manually operate the operation input 110 and the foot pedals 120 while viewing an affected area on the monitor 140. Commands can be provided to the remote operation apparatus 200 in accordance with these manual operations. Instructions provided to the remote operation apparatus 200 are transmitted to the surgical robot 100.

### (Foot Pedal)

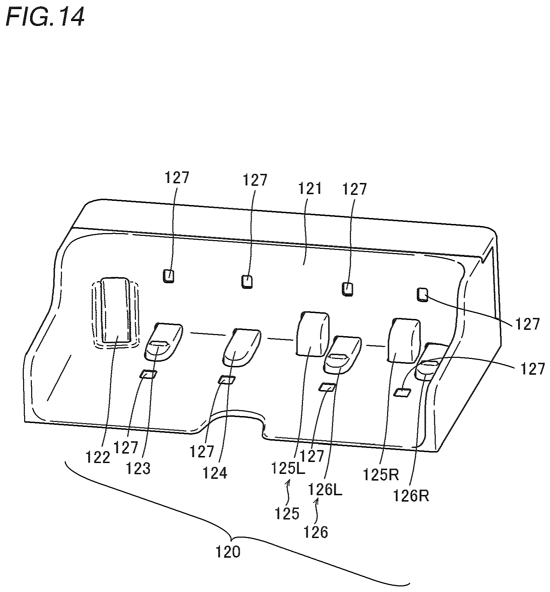

As shown in FIG. 14, a plurality of foot pedals 120 are configured to activate functions of the surgical instruments 1. The plurality of foot pedals 120 are provided in a base 121. The foot pedals 120 include a switching pedal 122, a clutch pedal 123, a camera pedal 124 , incision pedals 125, coagulation pedals 126, and foot detectors 127. The switching pedal 122, the clutch pedal 123, the camera pedal 124, the incision pedals 125, the coagulation pedals 126 are configured to be operated by the operator's foot. Also, the incision pedals 125 include an incision pedal 125R corresponding to a right-side robot arm 50 and an incision pedal 125L corresponding to a left-side robot arm 50. Also, the coagulation pedals 126 include a coagulation pedal 126R corresponding to a right-side robot arm 50 and a coagulation pedal 126L corresponding to a left-side robot arm 50.

The switching pedal 122 is configured to switch between the robot arms 50 to be operated by the operation input 110. The clutch pedal 123 is configured to activate a clutch function of temporarily halting operation connection between the robot arm 50 and the operation input 110. While the clutch pedal 123 is pressed by the operator, instructions provided by the operation input 110 are not transmitted to the robot arm 50. While the camera pedal 124 is pressed by the operator, the robot arm 50 that holds the endoscope 3 can be operated through the operation input 110. While the incision pedal 125 or the coagulation pedal 126 is pressed, an electric surgical apparatus is active.

### (Vision Unit and Image Processing Unit)

As shown in FIG. 1, a cart 210 holds a vision unit 300 and an image processing unit 400. The image processing unit 400 is configured to process images captured by the endoscope 3. A display 220 is arranged on the cart 210. The display 220 is configured to display images captured by the endoscope 3. Here, the display 220 is an example of a second display.

### (Configuration of Control System)

As shown in FIG. 15, the robotic surgical system 500 includes a first controller 310, an arm controller 320, a positioner controller 330, operation controllers 340 and a second controller 350. In addition, the robotic surgical system 500 includes a storage 311 connected to the first controller 310, and a storage 351 connected to the second controller 350.

The first controller 310 is accommodated in the medical cart 10, and configured to communicate with the arm controller 320 and the positioner controller 330 so that the robotic surgical system 500 is controlled as a whole. Specifically, the first controller 310 is configured to communicate with the arm controller 320, the positioner controller 330 and the operation controllers 340 and control these controllers. The first controller 310 is connected to the arm controller 320, the positioner controller 330 and the operation controllers 340 through LAN, or the like. The first controller 310 is arranged in the medical cart 10.

Each of the plurality of robot arms 50 includes the arm controller 320. In other words, a plurality of arm controllers 320 the number of which corresponds to the number of the plurality of robot arms 50 are included in the medical cart 10.

As shown in FIG. 15, the input device 22 is connected to the first controller 310 through LAN, or the like. The status indicator 41, the arm status indicator 42, the operating handle 23, the throttle grip 23a, and the joystick 22b are connected to the positioner controller 330 through a wiring line 360 by means of a communication network that can share information with them by using serial communication. Although all of the status indicator 41, arm status indicator 42, and the like are connected to each other through one wiring line 360 in FIG. 15, wiring lines 360 are actually provided to each of the status indicator 41, the arm status indicator 42, the operating handle 23, the throttle grip 23a, the joystick 22b, the stabilizer 24 and the electric cylinder 25.

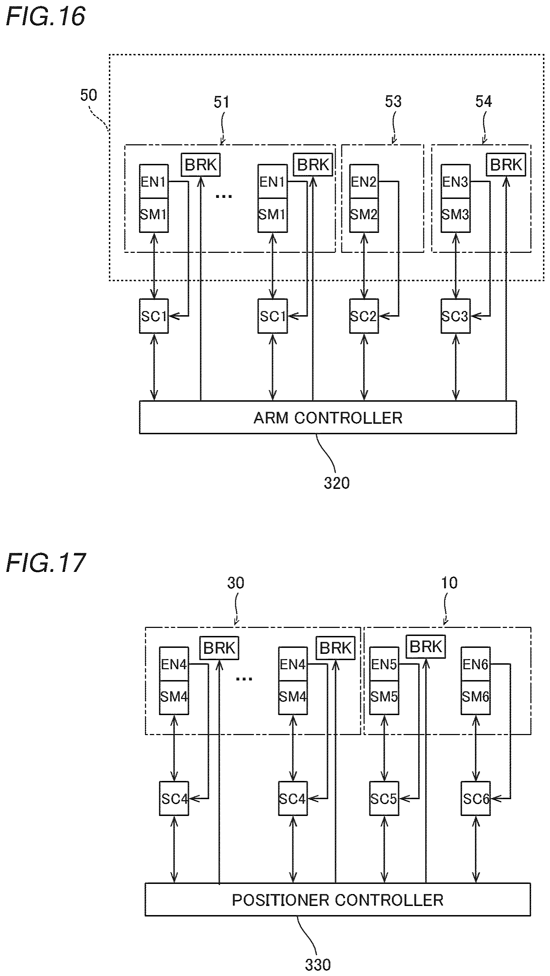

As shown in FIG. 16, each arm 51 includes a plurality of servomotors SM1, a plurality of encoders EN1 and a plurality of speed reducers corresponding to the Joints JT1, JT2, JT3, JT4, JT5, JT6 and JT7. The encoder EN1 is configured to detect a rotation angle of the servomotor SM1. The speed reducer is configured to reduce a rotation of the servomotor SM1 whereby increasing its torque. A servo controller SC1 is configured to control the servomotor SM1, and is arranged in the medical cart 10 adjacent to the arm controller 320. Also, the encoder EN1 is configured to detect the rotation angle of the servomotor SM1, and is electrically connected to the servo controller SC1.

The second link 53 includes a servomotor SM2 configured to rotate a driven member arranged in a driven unit 2a of the surgical instrument 1, an encoder EN2, and a speed reducer. The encoder EN2 is configured to detect a rotation angle of the servomotor SM2. The speed reducer is configured to reduce a rotation of the servomotor SM2 whereby increasing its torque. The medical cart 10 includes a servo controller SC2 configured to control the servomotor SM2 for driving the surgical instrument 1. The encoder EN2 for detecting the rotation angle of the servomotor SM2 is electrically connected to the servo controller SC2. Note that a plurality of servomotors SM2, a plurality of encoders EN2 and a plurality of servo controllers SC2 are included.

The translation mechanism 54 includes a servomotor SM3 configured to translationally move the surgical instrument 1, an encoder EN3, and a speed reducer. The encoder EN3 is configured to detect a rotation angle of the servomotor SM3. The speed reducer is configured to reduce a rotation of the servomotor SM3 whereby increasing its torque. The medical cart 10 includes a servo controller SC3 configured to control the servomotor SM3 for translationally moving the surgical instrument 1. The encoder EN3 for detecting the rotation angle of the servomotor SM3 is electrically connected to the servo controller SC3. Here, the encoders EN1, EN2, and EN3 may be, for example, magnetic, mechanical, electromagnetic induction type, or the like. Since the encoders EN1, EN2, and EN3 themselves have high resolution, positional accuracy of a distal end of the robot arm 50 and positional accuracy of the distal end of the surgical instrument 1 are also increased. Further, by reducing rotational speeds transmitted from the servomotors SM1, SM2, and SM3 by speed reducers, accuracy of a rotational angle of joints of the robot arm 50, which are output shafts, is further increased.

The first controller 310 is configured to generate instruction values that specify positions of the servomotor SM1, SM2 and SM3 in accordance with manual operation that is received by the remote operation apparatus 200, and to drive the servomotor SM1, SM2 and SM3 in accordance with the instruction values. If any of differences between instruction values and positions of servomotor SM1, SM2 and SM3 detected by sensors becomes greater than an allowable range, the first controller 310 determines an error of abnormal deviation.

As shown in FIG. 17, the positioner 30 includes a plurality of servomotors SM4, a plurality of encoders EN4 and a plurality of speed reducers corresponding to a plurality of joints 33 of the positioner 30. Each encoder EN4 is configured to detect a rotation angle of the servomotor SM4. The speed reducer is configured to reduce a rotation of the servomotor SM4 whereby increasing its torque.

The medical cart 10 includes wheels including front wheels as driving wheels, and rear wheels configured to be steered by manually operating the operating handle 23. The rear wheels are arranged closer to the operating handle 23 with respect to the front wheels. The medical cart 10 includes a servomotor SM5 configured to drive the front wheels of the medical cart 10, an encoder EN5, speed reducers, and brakes BRK. The speed reducer is configured to reduce a rotation of the servomotor SM5 whereby increasing its torque. Also, the operating handle 23 includes a potentiometer P1 shown in FIG. 3, and the servomotor SM5 of the front wheels can be driven in accordance with a rotation angle detected by the potentiometer P1 in response to a twisting amount of the throttle grip 23a. The rear wheels of the medical cart 10 have a twin-wheel type structure, and the rear wheels can be steered in accordance with a rightward/leftward turn of the operating handle 23. Also, the operating handle 23 includes a potentiometer P2 shown in FIG. 3 on a turning shaft, and the rear wheel of the medical cart 10 is provided with a servomotor SM6, an encoder EN6, and speed reducers. The speed reducer is configured to reduce a rotation of the servomotor SM6 whereby increasing its torque. The servomotor SM6 can be driven in accordance with a rotation angle detected by the potentiometer P2 in response to a rightward/leftward turning amount of the operating handle 23. In other words, power is assisted by the servomotor SM6 when the rear wheels are steered by turning the operating handle 23 rightward or leftward.

The medical cart 10 can be moved forward or rearward by driving the front wheels. Also, the medical cart 10 can be turned rightward or leftward by steering the rear wheels by turning the operating handle 23.

As shown in FIG. 17, the medical cart 10 includes servo controllers SC4 configured to control the servomotors SM4 for moving the positioner 30. Also, the encoder EN4 is configured to detect the rotation angle of the servomotor SM4, and is electrically connected to the servo controller SC4. The medical cart 10 includes a servo controller SC5 configured to control the servomotor SM5 for driving the front wheels of the medical cart 10. The encoder EN5 for detecting the rotation angle of the servomotor SM5 is electrically connected to the servo controller SC5. The medical cart 10 includes a servo controller SC6 configured to control the servomotor SM6 for power assistance to steering of the rear wheels of the medical cart 10. The encoder EN6 for detecting the rotation angle of the servomotor SM6 is electrically connected to the servo controller SC6.

As shown in FIGS. 16 and 17, the joints JT1, JT2, JT3, JT4, JT5, JT6 and JT7 of the arm 51, and the joints 33 of the positioner 30 include their brake BRK. Also, the front wheels of the medical cart 10, the arm base 40 and the translation mechanism 54 include their brake BRK. The arm controller 320 is configured to one-directionally transmit control signals to the brakes BRK of the joints JT1, JT2, JT3, JT4, JT5, JT6 and JT7 of the arm 51, and the translation mechanism 54. The control signals are configured to indicate on/off of the brakes BRK. The signals indicating on of the brakes BRK include a signal that instructs the brake BRK to keep activating. The control signals transmitted from the positioner controller 330 to the brakes BRK included in the joints 33 of the positioner 30 and the arm base 40 are configured similar to the control signals transmitted from the arm controller. On startup, all the brakes BRK of the arm base 40, the arm 51 and the translation mechanism 54 are turned off but the servomotors SM are driven to keep postures of the robot arm 50 and the arm base 40 against gravity. If an error occurs in the robotic surgical system 500, the brakes BRK included in the arm base 40, the arm 51 and the translation mechanism 54 are turned on. When the error in the robotic surgical system 500 is reset, the brakes BRK included in the arm base 40, the arm 51 and the translation mechanism 54 are turned off. When shutdown operation is performed in the robotic surgical system 500, the brakes BRK included in the arm base 40, the arm 51 and the translation mechanism 54 are turned on. The brakes BRK of the front wheels of the medical cart 10 are constantly turned on, and the brakes BRK are deactivated only when the enable switch 23b is kept pressed. Also, the brakes BRK of the joints 33 of the positioner 30 are constantly turned on, and the brakes BRK are deactivated only when the enable switch 22c is kept pressed.

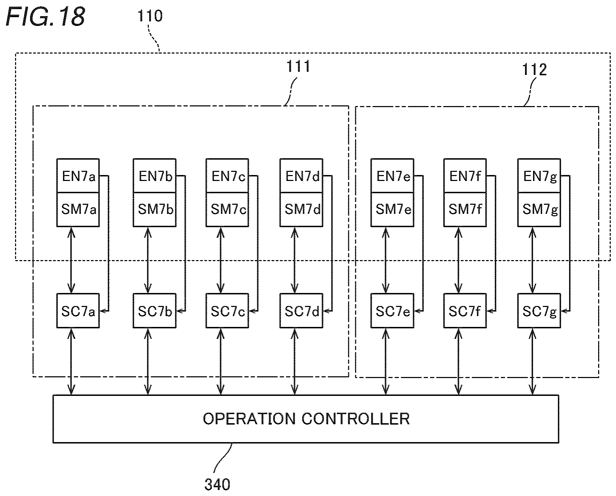

As shown in FIG. 18, the joints JT21, JT22, JT23, JT24, JT25, JT26 and JT27 of the operation input 110 include servomotors SM7a, SM7b, SM7c, SM7d, SM7e, SM7f and SM7g, respectively. The servomotor SM7a rotates the link 111a about the A21 axis. The servomotor SM7b rotates the link 111b about the A22 axis. The servomotor SM7c rotates the link 111c about the A23 axis. The servomotor SM7d rotates the link 112a about the A24 axis. The servomotor SM7e rotates the link 112b about the A25 axis. The servomotor SM7f rotates the link 112c about the A26 axis. The servomotor SM7g rotates the grip support 112d about the A27 axis. Further, servo controllers SC7a, SC7b, SC7c, SC7d, SC7e, SC7f and SC7g configured to control the servomotors are provided. Encoders EN7a, EN7b, EN7c, EN7d, EN7e, EN7f and EN7g for detecting rotation angles of the servomotors are electrically connected to the servo controllers. Each of the operation input 110L and the operation input 110R includes the servomotors, the servo controllers and the encoders.

The first controller 310 is configured to control the servomotors through the operation controllers 340 so that torques are produced to cancel out gravitational torques applied to the rotation axes of the servomotors in postures of the operation inputs 110. Accordingly, the operator can manually operate the operation inputs 110 by relatively small forces.

When the operator rotationally operates the grip support 112d about the A27 axis of the operation input 110 shown in FIGS. 12 and 13, the shaft 2d of the instrument 2 rotates about the A9 axis shown in FIG. 5. When the operator rotationally operates joints JT24, JT25, and JT26 of the operation input 110 shown in FIGS. 12 and 13, the end effector 2b pivots about the A10 axis or the A11 axis shown in FIG. 5.

The operation controllers 340 are provided in a main body of the remote operation apparatus 200. The operation controllers 340 are configured to control the operation inputs 110. The operation controllers 340 are associated with both the left-hand side operation input 110L and the right-hand side operation input 110R as shown in FIG. 15.

As shown in FIG. 15, the vision unit 300 and the image processing unit 400 are connected to the first controller 310 through LAN. The display 220 is connected to the vision unit 300.

### (Specification of Pivot Position)

An operation for specifying pivot positions PP for four robot arms 50 is described. As shown in FIG. 15, a program 311a including processes from step S1 to step S9 described below is stored in the storage 311. The program 311a may be stored in the storage 351. Further, pivot-position specifying tools 4 are attached in advance to the robot arms 50a, 50b, and 50d. The endoscope 3 is attached in advance to the robot arm 50c. Here, the trocar T is inserted in advance through a body surface S into a body of a patient P. Hereinafter, the pivot position of the endoscope 3 is referred to as PP1, and the pivot position of the instrument 2 is referred to as PP2. The pivot position PP1 and the pivot position PP2 are collectively referred to as a pivot position PP.

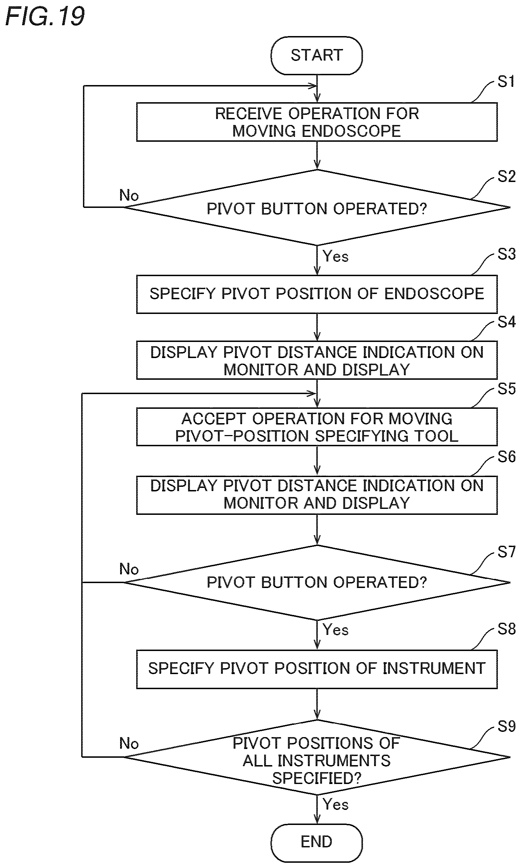

As shown in FIG. 19, in step S1, the arm operation input 60 receives an operation for moving the endoscope 3. For example, when the joystick 62 is operated by the operator, the endoscope 3 is moved outside the trocar T.

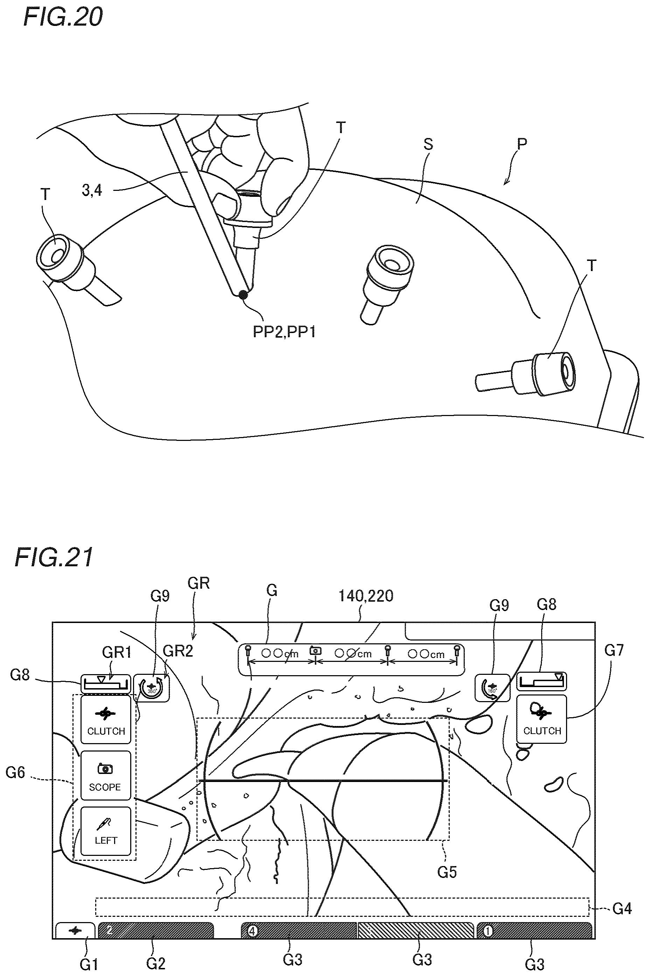

In step S2, the second controller 350 determines whether or not the pivot button 66 is operated. For example, as shown in FIG. 20, the operator first moves the robot arm 50c by operating the arm operation input 60 to move the distal end of the endoscope 3, which is attached to the distal end side of the robot arm 50c, to an insertion position of the trocar T inserted through the body surface S into the body of the patient P, and then operates the pivot button 66 so that the second controller 350 stores the position of the distal end of the endoscope 3 as a pivot position PP1 in the storage 351 in step S3. The processes of steps S1 and S2 are repeated until the pivot button 66 is operated.

In step S4, in this embodiment, as shown in FIG. 21, processing of displaying, on the monitor 140 and the display 220, a graphical user interface including a pivot distance indication G representing a distance from a preset reference point to a predetermined portion of the surgical instrument 1 is executed. As shown in FIG. 1, the monitor 140 is a display that is included in the remote operation apparatus 200 and is visually recognized by the operator who operates the remote operation apparatus 200. The display 220 is a display that is arranged separately from the surgical robot 100 and the remote operation apparatus 200. Here, the vision unit 300 is an example of a controller. Further, the pivot distance indication G is an example of a distance indication.

As shown in FIG. 21, the pivot distance indication G is displayed so as to be superimposed on an image GR captured by the endoscope 3. In addition to the pivot distance indication G, on the monitor 140 and the display 220, a state of the clutch pedal 123 is indicated in a clutch area G1, and a state of the camera pedal 124 is displayed in a camera area G2. Further, in hand areas G3, states of the respective robot arms 50 and states of the coagulation pedal 126 and the incision pedal 125 are displayed. A medical instrument area G4 indicates current usage/maximum usage numbers of the surgical instruments 1 attached to the robot arms 50. A level area G5 indicates information on an angle of the endoscope 3. A left pop-up area G6 indicates icons in a hovering position in which the foot of the operator is placed on the foot pedal 120. A right pop-up area G7 indicates an icon when feet of the operator are placed on the coagulation pedal 126R and the incision pedal 125R. In a first area G8, a first graphical indication GR1 indicating a movable range of the robot arm 50, and an operational movable range that is allowed for the operation input 110 to move the robot within the movable range of the robot arm 50 are displayed. In a second area G9, a second graphical indication GR2 indicating a movable range of the robot arm 50, and an operational movable range that is allowed for the operation input 110 to move the robot within the movable range of the robot arm 50 are displayed.

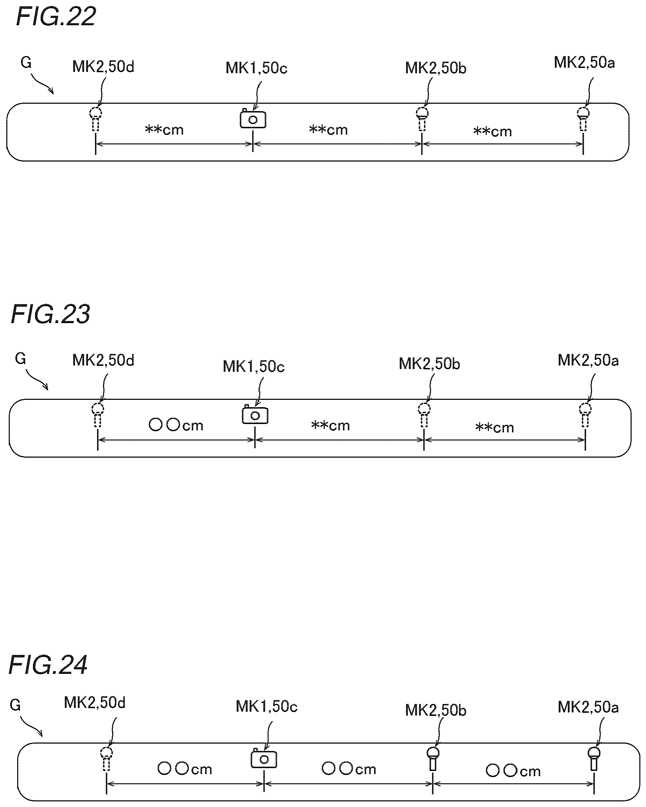

Further, in this embodiment, as shown in FIG. 22, the pivot distance indication G includes a first marker MK1 corresponding to the robot arm 50c and second markers MK2 corresponding to the robot arms 50a, 50b, and 50d. The first marker MK1 has, for example, a camera shape. The second marker MK2 has, for example, a trocar T shape. Three second markers MK2 are displayed so as to correspond to the robot arms 50a, 50b, and 50d. Further, the pivot distance indication G includes numerical values of distances from the pivot position PP1 of the endoscope 3 to predetermined portions of the pivot-position specifying tools 4 that are displayed between the first marker MK1 and the second markers MK2. Further, the first marker MK1 is in a lit state. Further, in FIG. 22, the first marker MK1 in the lit state is represented by a solid line.

Further, in this embodiment, the second markers MK2 have different indication forms before and after the pivot position PP2 is specified. For example, the second markers MK2 blink before the pivot position PP2 is specified, and are lit after the pivot position PP2 is specified. At the stage of step S4, the second markers MK2 are blinking. Further, in FIG. 22, the second markers MK2 in a blinking state are represented by broken lines.

In step S5, the arm operation input 60 receives an operation for moving the pivot-position specifying tool 4. For example, when the operator operates the joystick 62, the pivot-position specifying tool 4 is moved outside the trocar T.

In step S6, in this embodiment, as shown in FIG. 23, while the pivot-position specifying tool 4 is being moved to a desired position by the arm operation input 60, the vision unit 300 executes processing of displaying, on the monitor 140 and the display 220, a graphical user interface including a pivot distance indication G representing a distance from a preset reference point to a predetermined portion of the pivot-position specifying tool 4. Specifically, the vision unit 300 executes processing of displaying, on the monitor 140 and the display 220, as the pivot distance indication G, a distance from the pivot position PP1 of the endoscope 3 preset as the reference point to the predetermined portion of the pivot-position specifying tool 4. Further, in this embodiment, the predetermined portion of the pivot-position specifying tool 4 is a distal end of the pivot-position specifying tool 4. In FIG. 23, a distance from the pivot position PP1 of the endoscope 3 attached to the robot arm 50c to the distal end of the pivot-position specifying tool 4 attached to the robot arm 50d is indicated in units of cm. The distance from the pivot position PP1 of the endoscope 3 to the distal end of the pivot-position specifying tool 4 corresponds to a distance between the trocars T, and, for example, a distance of about 8 cm is recommended. Note that the recommended distance of about 8 cm is merely an example, and is not limited thereto. For example, the distance between the trocars T differs depending on a body shape of the patient P.

Here, the first controller 310 recognizes an overall posture of the surgical robot 100. Accordingly, the first controller 310 recognizes coordinates of the distal end of the pivot-position specifying tool 4. Further, the first controller 310 acquires coordinates of the pivot position PP1 of the endoscope 3 from the second controller 350. Then, the first controller 310 acquires a distance from the pivot position PP1 of the endoscope 3 to the distal end of the pivot-position specifying tool 4 based on the coordinates of the distal end of the pivot-position specifying tool 4 and the coordinates of the pivot position PP1 of the endoscope 3 acquired from the second controller 350. The vision unit 300 acquires this distance from the first controller 310 and executes processing of displaying the distance on the monitor 140 and the display 220. Further, position accuracy of the coordinates of the distal end of the pivot-position specifying tool 4 is, for example, within ±5 mm.

Further, the robot arm 50d and the robot arm 50b to which the pivot-position specifying tools 4 are respectively attached are arranged adjacent to the robot arm 50c to which the endoscope 3 is attached;;;; Accordingly, when the pivot position PP2 is being specified for the instrument 2 attached to each of the robot arm 50d and the robot arm 50b, a distance from the pivot position PP1 of the endoscope 3 to the distal end of the pivot-position specifying tool 4 is indicated. On the other hand, the robot arm 50a to which the pivot-position specifying tool 4 is attached is arranged adjacent to the robot arm 50b, but is not arranged adjacent to the robot arm 50c to which the endoscope 3 is attached. Accordingly, in this embodiment, when the pivot position PP2 is being specified for the instrument 2 attached to the robot arm 50a, a distance from the distal end of the pivot-position specifying tool 4 attached to the robot arm 50b, that is, from the pivot position PP2 of the pivot-position specifying tool 4 attached to the robot arm 50b to the distal end of the pivot-position specifying tool 4 attached to the robot arm 50a is indicated on the monitor 140 and the display 220 as the pivot distance indication G.

Further, in this embodiment, the vision unit 300 executes processing of displaying, on the monitor 140 and the display 220, a distance from the pivot position PP1 of the endoscope 3 as the reference point to a distal end as the predetermined portion of the pivot-position specifying tool 4 in real time. That is, while the pivot-position specifying tool 4 is being moved to a desired position by the arm operation input 60, the distance indicated on the monitor 140 and the display 220 changes in real time.

As described above, in this embodiment, the vision unit 300 executes processing of displaying, on the monitor 140 and the display 220, the pivot distance indication G before the pivot position PP2 is stored in the storage 351 by operation of the pivot button 66. That is, in step S8, the pivot distance indication G is displayed on the monitor 140 and the display 220 before the pivot position PP2 is stored in the storage 351.

In step S7, the second controller 350 determines whether or not the pivot button 66 is operated. For example, the operator first moves the robot arm 50d by operating the arm operation input 60 to move the distal end of the pivot-position specifying tool 4, which is attached to the distal end side of the robot arm 50d, to the insertion position of the trocar T inserted through the body surface S into the body of the patient P, and then operates the pivot button 66 so that the second controller 350 stores the position of the distal end of the pivot-position specifying tool 4 as a pivot position PP2 of the instrument 2 in the storage 351 in step S8. Then, the second marker MK2 for the robot arm 50d changes from a blinking state to a lit state. The processes of steps S5 to S7 are repeated until the pivot button 66 is operated.

In step S9, the first controller 310 determines whether or not the pivot positions PP2 are specified for all of the instruments 2 to be attached to the robot arms 50a, 50b, and 50d. If the determination in step S9 is No, the process returns to step S5. If the pivot positions PP2 are specified for all of the instruments 2, as shown in FIG. 24, all of the second markers MK2 become in a lit state. The pivot distance indication G continues to be displayed until a following operation is performed. The following movement is movement of the robot arm 50 by the operator by operating the operation input 110. Further, even before the pivot positions PP2 are specified for all of the robot arms 50a, 50b, and 50d, if the following operation is started, the pivot distance indication G disappears.

### [Advantages of the Embodiment]

While the surgical instrument 1 is being moved to a desired position by the arm operation input 60, the vision unit 300 executes processing of displaying, on the monitor 140 and the display 220, a graphical user interface including a pivot distance indication G representing a distance from a preset reference point to a predetermined portion of the surgical instrument 1. Accordingly, while the surgical instrument 1 is being moved to a desired position by the arm operation input 60, a pivot distance indication G representing a distance from a preset reference point to a predetermined portion of the surgical instrument 1 is displayed, so that an operator can recognize the distance from the reference point to the predetermined portion of each surgical instrument 1. That is, the operator can specify the pivot position PP2 based on the distance from the reference point. As a result, the pivot positions PP of the surgical instruments 1 respectively attached to the plurality of robot arms 50 can be appropriately specified.

The plurality of robot arms 50 include the robot arm 50c having a distal end to which the endoscope 3 as the surgical instrument 1 is attached, and a plurality of robot arms 50a, 50b and 50d each having a distal end to which the pivot-position specifying tool 4 as the surgical instrument 1 is attached. The vision unit 300 executes processing of displaying, on the monitor 140 and the display 220, as the pivot distance indication G, a distance from the pivot position PP1 of the endoscope 3 preset as the reference point to the predetermined portion of the pivot-position specifying tool 4. Accordingly, since an operator can recognize a distance between the pivot position PP1 of the endoscope 3 and a predetermined portion of the pivot-position specifying tool 4, the operator can appropriately specify the pivot position PP2 of the instrument 2 by using the pivot-position specifying tool 4.

The plurality of robot arms 50a, 50b, and 50d include adjacent robot arms 50b and 50d arranged adjacent to the robot arm 50c to which the endoscope 3 is attached, and an end robot arm 50a arranged adjacent to the robot arm 50b to which the pivot-position specifying tool 4 is attached. The vision unit 300 is configured to execute processing of displaying, on the monitor 140 and the display 220, a distance from the pivot position of the endoscope 3 to a predetermined portion of the pivot-position specifying tool 4 attached to the robot arms 50b and 50d, and a distance from the predetermined portion of the pivot-position specifying tool 4 attached to the robot arm 50b to a predetermined portion of the pivot-position specifying tool 4 attached to the robot arm 50a, as the pivot distance indication G. Accordingly, since the operator can recognize a distance between pivot positions PP of robot arms 50 adjacent to each other, the operator can specify the pivot positions PP while avoiding interference between the surgical instruments 1 attached to the robot arms 50 adjacent to each other.

The pivot distance indication G includes the first marker MK1 corresponding to the robot arm 50c, the second markers MK2 corresponding to the robot arms 50a, 50b and 50d, and numerical values of distances from the pivot position PP1 of the endoscope 3 to predetermined portions of the pivot-position specifying tools 4 that are displayed between the first marker MK1 and the second markers MK2. Accordingly, the operator can intuitively recognize a distance between pivot positions PP by using the first marker MK1, the second markers MK2, and numerical values of the distances.

The second markers MK2 have different indication forms before and after the pivot position PP2 is specified. Accordingly, the operator can intuitively recognize whether or not the pivot position PP2 has been specified for a desired robot arm 50 based on the indication form.

The vision unit 300 is configured to execute processing of displaying, in real time, on the monitor 140 and the display 220, a distance from the reference point to a predetermined portion of the surgical instrument 1. Accordingly, since the operator can recognize a distance between pivot positions PP in real time, the operator can easily specify the pivot positions at desired positions.

The vision unit 300 is configured to execute processing of displaying, on the monitor 140 and the display 220, a distance from the reference point to the distal end of the surgical instrument 1 as the predetermined portion. Accordingly, since the pivot position PP is often specified near the distal end of the surgical instrument 1, by displaying a distance from the reference point to the distal end of the surgical instrument 1, the pivot position can be more appropriately specified.

The robotic surgical system 500 is equipped with a surgical robot 100 in which a plurality of robot arms 50 are arranged, and a remote operation apparatus 200 that operates the surgical robot 100. As a display according to the present disclosure, the monitor 140 that is included in the remote operation apparatus 200 and is visually recognized by the operator who operates the remote operation apparatus 200, and the display 220 that is arranged separately from the surgical robot 100 and the remote operation apparatus 200 are provided. Accordingly, since the pivot distance indication G is displayed on both the monitor 140 and the display 220, the operator can specify the pivot position PP while viewing either the monitor 140 or the display 220.

The vision unit 300 executes processing of displaying, on the monitor 140 and the display 220, the pivot distance indication G before the pivot position PP2 is stored in the storage 351 by operation of the pivot button 66. Accordingly, since the operator can view the pivot distance indication G while specifying the pivot position PP2, the operator can more easily specify the pivot position PP.

### [Modified Embodiments]

Note that the embodiment disclosed this time must be considered as illustrative in all points and not restrictive. The scope of the present disclosure is not shown by the above description of the embodiments but by the scope of claims for patent, and all modifications or modified examples within the meaning and scope equivalent to the scope of claims for patent are further included.

While the example in which the reference point of the present disclosure is set as a preset pivot position PP1 of the endoscope 3 has been shown in the aforementioned embodiment, the present disclosure is not limited to this. For example, a point other than the pivot position PP1 of the endoscope 3 may be set as the reference point. In this case, even before the pivot position PP1 of the endoscope 3 is set, a distance between the reference point and the distal end of the endoscope 3 is displayed in the pivot distance indication G. Further, when the pivot position PP1 of the pivot-position specifying tool 4 is set before that of the endoscope 3, a distance from the previously set pivot position PP1 is displayed in the pivot distance indication G.

While the example in which, as the pivot distance indication G, a distance from the preset pivot position PP1 of the endoscope 3 to the distal end of the pivot-position specifying tool 4 is displayed has been shown in the aforementioned embodiment, the present disclosure is not limited to this. For example, as the pivot distance indication G, a distance from the preset pivot position PP1 of the endoscope 3 to a part other than the distal end of the pivot-position specifying tool 4 may be displayed.

While the example in which a distance from the distal end of the pivot-position specifying tool 4 attached to the robot arm 50b adjacent to the endoscope 3 to the distal end of the pivot-position specifying tool 4 attached to the robot arm 50a not adjacent to the endoscope 3 is displayed has been shown in the aforementioned embodiment, the present disclosure is not limited to this. For example, also for the robot arm 50a not adjacent to the endoscope 3, a distance from the distal end of the endoscope 3 to the distal end of the pivot-position specifying tool 4 attached to the robot arm 50a may be displayed.

While the example in which the first marker MK1 has a camera shape, and the second marker MK2 has a trocar T shape has been shown in the aforementioned embodiment, the present disclosure is not limited to this. In the present disclosure, the first marker MK1 may have a shape other than the camera shape, and the second marker MK2 may have a shape other than the trocar T shape.

While the example in which a distance from the pivot position PP1 of the endoscope 3 to the distal end of the pivot-position specifying tool 4, and a distance between the distal ends of the pivot-position specifying tools 4 are indicated in units of cm has been shown in the aforementioned embodiment, the present disclosure is not limited to this. For example, these distances may be indicated in mm.

While the example in which the second marker MK2 blinks before the pivot position PP2 is specified and is lit after the pivot position PP2 is specified has been shown in the aforementioned embodiment, the present disclosure is not limited to this. For example, the second marker MK2 may be displayed in different colors before and after the pivot position PP2 is specified.

While the example in which a distance from the pivot position PP1 of the endoscope 3 to the distal end of the pivot-position specifying tool 4, and a distance between the distal ends of the pivot-position specifying tools 4 are displayed in real time, that is, so as to continuously change has been shown in the aforementioned embodiment, the present disclosure is not limited to this. For example, these distances may be updated at predetermined time intervals.

While the example in which the pivot distance indication G is displayed on both the monitor 140 arranged on the remote operation apparatus 200 and the display 220 arranged on the cart 210 has been shown in the aforementioned embodiment, the present disclosure is not limited to this. In the present disclosure, the pivot distance indication G may be displayed on only one of the monitor 140 and the display 220.

While an example in which the pivot distance indication G is displayed when the pivot position PP2 is initially specified has been shown in the aforementioned embodiment, the present disclosure is not limited to this. For example, after the pivot position PP2 of the instrument 2 has been specified once, the vision unit 300 may execute processing of displaying the pivot distance indication G on the monitor 140 and the display 220 in a pivot position change mode for changing the pivot position PP2. For example, after the pivot position PP2 has been specified once, when the pivot button 66 is pressed twice consecutively, the robotic surgical system 500 enters the pivot position change mode. Then, the distal end of the pivot-position specifying tool 4 is moved by operating the robot arm 50a, 50b, or 50d by the arm operation input 60, and the pivot position PP2 is changed by pressing the pivot button 66 once at the moved position in the pivot position change mode. Also, the pivot distance indication G is displayed on the monitor 140 and the display 220 in the pivot position change mode. Accordingly, the vision unit 300 executes processing of displaying the pivot distance indication G on the monitor 140 and the display 220 in the pivot position change mode for changing the pivot position PP after the pivot position PP2 serving as a pivot point for movement of the instrument 2 has been specified once. As a result, since the pivot distance indication G is displayed also when the pivot position PP2 is changed, the pivot position PP2 can be easily changed.

Further, in the present disclosure, the second controller 350 may be configured to issue a warning when a distance from the reference point to the distal end serving as a predetermined portion of the pivot-position specifying tool 4 becomes equal to or less than a predetermined threshold. For example, the predetermined threshold is set so as to prevent the endoscope 3 and the pivot-position specifying tool 4 from colliding with each other. Further, the warning is, for example, an alarm sound or a warning in the form of characters displayed on the monitor 140 and the display 220. As described above, the second controller 350 issues a warning when the distance from the reference point to the predetermined portion of the surgical instrument 1 becomes equal to or less than the predetermined threshold. Accordingly, it is possible to prevent specification of the pivot position PP in a state in which the surgical instruments 1 are relatively close to each other.

While an example in which four trocars T are inserted through the body surface S into the body of the patient P in advance before the pivot position PP is specified has been shown in the aforementioned embodiment, the present disclosure is not limited to this. For example, among the four trocars T, before the pivot position PP is specified, the trocar T into which the endoscope 3 is inserted may be inserted through the body surface S into the body of the patient P, while the three trocars T into which the instruments 2 are inserted may not be inserted through the body surface S into the body of the patient P. Then, the pivot position PP2 is specified by operating the pivot button 66 in a state in which the distal end of the pivot-position specifying tool 4 attached to the distal end side of the robot arm 50a, 50b, or 50d has been moved to a desired position near the body surface S of the patient P. Here, although the pivot position PP2 cannot be specified at a position corresponding to the inside of the cylindrical trocars T in a state in which the trocars T are inserted through the body surface S into the body of the patient P, the pivot position PP2 can be specified at such positions since the three trocars T into which the instruments 2 are inserted are not inserted through the body surface S into the body of the patient P. As a result, the distances between the pivot positions PP determined during surgical planning can be accurately reproduced, so that an operating range of the surgical robot 100 can be set to an optimal region. In addition, while the pivot position PP2 of the instrument 2 is being specified, not only the distance between the pivot position PP2 and the pivot position PP1 of the endoscope 3 but also, for example, a direction in which the pivot-position specifying tool 4 is oriented with respect to a reference position such as a position in a longitudinal direction of the arm base 40 may be displayed on the monitor 140 and the display 220.

While the example in which four robot arms 50 are provided has been shown in the aforementioned embodiment, the present disclosure is not limited to this. In the present disclosure, the number of the robot arms 50 is not limited to four.

While the example in which the arms 51 and the positioner 30 are constructed of a 7-axis multi-joint robot has been shown in the aforementioned embodiment, the present disclosure is not limited to this. For example, the arms 51 and the positioner 30 are constructed of a multi-joint robot having an axis configuration other than the 7-axis multi-joint robot. The multi-joint robot having an axis configuration other than the 7-axis multi-joint robot can be a 6-axis or 8-axis multi-joint robot, for example.

While the example in which the surgical robot 100 includes the medical cart 10, the positioner 30, the arm base 40 and the robot arms 50 has been shown in the aforementioned embodiment, the present disclosure is not limited to this. The medical cart 10, the positioner 30 and the arm base 40 are not necessarily provided, and the surgical robot 100 may include only the robot arms 50, for example.

Functions of elements disclosed in the present specification may be implemented using one or more circuits or processing circuits including a general-purpose processor, a special-purpose processor, an integrated circuit, ASICs (Application Specific Integrated Circuits), FPGAs (Field Programmable Gate Arrays), and/or conventional circuits. The functions of the elements disclosed in the present specification may be implemented using one or more circuits or processing circuits including a combination of a general-purpose processor, a special-purpose processor, an integrated circuit, ASICs, FPGAs, and conventional circuits. The one or more circuits or processing circuits may be programmed using one or more programs stored together or separately in one or more memories, or may be configured to perform the disclosed functions. Since a processor includes transistors and other circuits, the processor is regarded as a processing circuit or a circuit. The processor may be a programmed processor configured to execute a program stored in a memory. In the present disclosure, a circuit, unit, or means is hardware that performs the recited functions alone or in combination with each other, or is hardware programmed to perform the recited functions alone or in combination with each other. The hardware may be any hardware disclosed in the present specification, as long as it is programmed or configured to perform the recited functions. A computer program including computer instructions is stored in a memory. The computer instructions provide logic and routines that allow the hardware to perform the methods disclosed in the present specification. The hardware includes, for example, a processing circuit or a circuit. The computer program may be implemented in a known format in a computer-readable storage medium, a computer program product, a memory device, a recording medium such as a CD-ROM or a DVD, and/or a memory of FPGAs or ASICs.

### [Embodiments]

The aforementioned exemplary embodiment will be understood as concrete examples of the following Embodiments by those skilled in the art.

### (Embodiment 1)

A robotic surgical system includes a plurality of robot arms each having a distal end to which a surgical instrument is attached; an operation input configured to operate movement of the surgical instrument by the robot arms; a pivot-position specifier configured to store, in a storage, a pivot position serving as a pivot point for movement of the surgical instrument when operated after a distal end of the surgical instrument has been moved to a desired position by the operation input; and a controller configured to execute processing of displaying, on a display, a graphical user interface including a distance indication representing a distance from a preset reference point to a predetermined portion of the surgical instrument while the surgical instrument is being moved to the desired position by the operation input.

### (Embodiment 2)

In the robotic surgical system according to Embodiment 1, the plurality of robot arms include a first robot arm having a distal end to which an endoscope as the surgical instrument is attached, and a plurality of second robot arms each having a distal end to which a pivot-position specifying tool as the surgical instrument is attached; and the controller is configured to execute processing of displaying, on the display, as the distance indication, a distance from a pivot position of the endoscope preset as the reference point to the predetermined portion of the pivot-position specifying tool.

### (Embodiment 3)

In the robotic surgical system according to Embodiment 2, the plurality of second robot arms include an adjacent robot arm arranged adjacent to the first robot arm, and an end robot arm arranged adjacent to the adjacent robot arm; and the controller is configured to execute processing of displaying, on the display, a distance from the pivot position of the endoscope to the predetermined portion of the pivot-position specifying tool attached to the adjacent robot arm, and a distance from the predetermined portion of the pivot-position specifying tool attached to the adjacent robot arm to the predetermined portion of the pivot-position specifying tool attached to the end robot arm as the distance indication.

### (Embodiment 4)

In the robotic surgical system according to Embodiment 2 or 3, the distance indication includes a first marker corresponding to the first robot arm, second markers corresponding to the second robot arms, and numerical values indicated between the first marker and the second markers and representing distances from the pivot position of the endoscope to the predetermined portions of the pivot-position specifying tools.

### (Embodiment 5)

In the robotic surgical system according to Embodiment 4, the second markers have different indication forms before and after the pivot position is specified.

### (Embodiment 6)

In the robotic surgical system according to any of Embodiments 1 to 5, the controller is configured to execute processing of displaying, in real time, on the display, a distance from the reference point to the predetermined portion of the surgical instrument.

### (Embodiment 7)

In the robotic surgical system according to any of Embodiments 1 to 6, the controller is configured to execute processing of displaying, on the display, a distance from the reference point to the distal end of the surgical instrument as the predetermined portion.

### (Embodiment 8)

In the robotic surgical system according to any of Embodiments 1 to 7, a surgical robot in which the plurality of robot arms are arranged, and an operation apparatus configured to operate the surgical robot are provided; and the display includes a first display included in the operation apparatus and visually recognized by an operator who operates the operation apparatus, and a second display arranged separately from the surgical robot and the operation apparatus.

### (Embodiment 9)

In the robotic surgical system according to any of Embodiments 1 to 8, the controller is configured to execute processing of displaying, on the display, the distance indication before the pivot position is stored in the storage by operation of the pivot-position specifier.

### (Embodiment 10)

In the robotic surgical system according to any of Embodiments 1 to 9, the controller is configured to execute processing of displaying, on the display, the distance indication in a pivot position change mode for changing the pivot position after the pivot position serving as the pivot point for movement of the surgical instrument has been specified once.

### (Embodiment 11)

In the robotic surgical system according to any of Embodiments 1 to 10, the controller is configured to issue a warning when a distance from the reference point to the predetermined portion of the surgical instrument becomes equal to or less than a predetermined threshold.

### (Embodiment 12)

A method controls a robotic surgical system, the robotic surgical system including a plurality of robot arms each having a distal end to which a surgical instrument is attached, an operation input configured to operate movement of the surgical instrument by the robot arms, and a pivot-position specifier configured to store, in a storage, a pivot position serving as a pivot point for movement of the surgical instrument when operated after a distal end of the surgical instrument has been moved to a desired position by the operation input, the method including receiving an operation for moving the surgical instrument by the operation input; and displaying, on a display, a graphical user interface including a distance indication representing a distance from a preset reference point to a predetermined portion of the surgical instrument while the surgical instrument is being moved to the desired position by the operation input.

### (Embodiment 13)

A program causes a computer to execute the method for controlling a robotic surgical system according to Embodiment 12.

### (Embodiment 14)

A storage medium stores the program for the method for controlling a robotic surgical system according to Embodiment 13.

## Claims

1. A robotic surgical system (500) comprising:
a plurality of robot arms (50) each having a distal end to which a surgical instrument (1) is attached;
an operation input (60) configured to operate movement of the surgical instrument by the robot arms;
a pivot-position specifier (66) configured to store, in a storage (351), a pivot position (PP) serving as a pivot point for movement of the surgical instrument when operated after a distal end of the surgical instrument has been moved to a desired position by the operation input; and
a controller (300) configured to execute processing of displaying, on a display (140, 220), a graphical user interface including a distance indication (G) representing a distance from a preset reference point (PP1) to a predetermined portion of the surgical instrument while the surgical instrument is being moved to the desired position by the operation input.

2. The robotic surgical system according to claim 1, wherein
the plurality of robot arms include
a first robot arm (50c) having a distal end to which an endoscope (3) as the surgical instrument is attached, and
a plurality of second robot arms (50a, 50b, 50d) each having a distal end to which a pivot-position specifying tool (4) as the surgical instrument is attached; and
the controller is configured to execute processing of displaying, on the display, as the distance indication, a distance from a pivot position (PP1) of the endoscope preset as the reference point to the predetermined portion of the pivot-position specifying tool.

3. The robotic surgical system according to claim 2, wherein
the plurality of second robot arms include an adjacent robot arm (50b, 50d) arranged adjacent to the first robot arm, and an end robot arm (50a) arranged adjacent to the adjacent robot arm; and
the controller is configured to execute processing of displaying, on the display,
a distance from the pivot position of the endoscope to the predetermined portion of the pivot-position specifying tool attached to the adjacent robot arm, and
a distance from the predetermined portion of the pivot-position specifying tool attached to the adjacent robot arm to the predetermined portion of the pivot-position specifying tool attached to the end robot arm as the distance indication.

4. The robotic surgical system according to claim 2 or 3, wherein
the distance indication includes
a first marker (MK1) corresponding to the first robot arm,
second markers (MK2) corresponding to the second robot arms, and
numerical values indicated between the first marker and the second markers and representing distances from the pivot position of the endoscope to the predetermined portions of the pivot-position specifying tools.

5. The robotic surgical system according to claim 4, wherein the second markers have different indication forms before and after the pivot position is specified.

6. The robotic surgical system according to any of claims 1 to 5, wherein the controller is configured to execute processing of displaying, in real time, on the display, a distance from the reference point to the predetermined portion of the surgical instrument.

7. The robotic surgical system according to any of claims 1 to 6, wherein the controller is configured to execute processing of displaying, on the display, a distance from the reference point to the distal end of the surgical instrument as the predetermined portion.

8. The robotic surgical system according to any of claims 1 to 7 comprising
a surgical robot (100) in which the plurality of robot arms are arranged, and
an operation apparatus (200) configured to operate the surgical robot, wherein
the display includes
a first display (140) included in the operation apparatus and visually recognized by an operator who operates the operation apparatus, and
a second display (220) arranged separately from the surgical robot and the operation apparatus.

9. The robotic surgical system according to any of claims 1 to 8, wherein the controller is configured to execute processing of displaying, on the display, the distance indication before the pivot position is stored in the storage by operation of the pivot-position specifier.

10. The robotic surgical system according to any of claims 1 to 9, wherein the controller is configured to execute processing of displaying, on the display, the distance indication in a pivot position change mode for changing the pivot position after the pivot position serving as the pivot point for movement of the surgical instrument has been specified once.

11. The robotic surgical system according to any of claims 1 to 10, wherein the controller is configured to issue a warning when a distance from the reference point to the predetermined portion of the surgical instrument becomes equal to or less than a predetermined threshold.

12. A method for controlling a robotic surgical system (500), the robotic surgical system including a plurality of robot arms (50) each having a distal end to which a surgical instrument (1) is attached, an operation input (60) configured to operate movement of the surgical instrument by the robot arms, and a pivot-position specifier (66) configured to store, in a storage (351), a pivot position (PP) serving as a pivot point for movement of the surgical instrument when operated after a distal end of the surgical instrument has been moved to a desired position by the operation input,
the method comprising:
receiving an operation for moving the surgical instrument by the operation input; and
displaying, on a display (140, 220), a graphical user interface including a distance indication (G) representing a distance from a preset reference point (PP1) to a predetermined portion of the surgical instrument while the surgical instrument is being moved to the desired position by the operation input.

13. A program (311a) for causing a computer to execute the method for controlling a robotic surgical system according to claim 12.

14. A storage medium (311) storing the program for the method for controlling a robotic surgical system according to claim 13.
